**Europäisches Patentamt**

**European Patent Office**

**Office européen des brevets**

(19)

(11) Veröffentlichungsnummer: **0 127 769**
**B1**

(12)

# EUROPÄISCHE PATENTSCHRIFT

(45) Veröffentlichungstag der Patentschrift:
24.09.86

(51) Int. Cl.⁴: **C 12 M 1/00**

(21) Anmeldenummer: **84104890.3**

(22) Anmeldetag: **02.05.84**

(54) Anlage mit aus synthetischer Persenning hergestellten Behältern zur schrittweisen (aeroben und anaeroben) Gärung von tierischem Streumist und landwirtschaftlichen und/oder lebensmittelindustriellen Nebenprodukten.

(30) Priorität: **02.05.83 HU 149183**

(43) Veröffentlichungstag der Anmeldung:
**12.12.84 Patentblatt 84/50**

(45) Bekanntmachung des Hinweises auf die Patenterteilung:
**24.09.86 Patentblatt 86/39**

(84) Benannte Vertragsstaaten:
**AT BE CH DE FR GB IT LI LU NL SE**

(56) Entgegenhaltungen:

(73) Patentinhaber: **ENERGIAGAZDALKODASI INTEZET, 33- 34, Bem- rakpart, H-1027 Budapest II (HU)**
Patentinhaber: **Rozmaring Fejlesztési Innovációs Mezögazdasági Szakcsoport, H-2083 Solymár (HU)**
Patentinhaber: **GRABOPLAST Pamutszövö és Mübörgyár, Fehérvari ut 16/B, H-9002 Györ (HU)**
Patentinhaber: **Kisbéri "Virágzo" Mezögazdasági Termelöszövetkezet, H-2870 Kisbér (HU)**

(72) Erfinder: **Bartha, István, Dr. Dipl.- Ing., Vaci ut 41, H-1056 Budapest (HU)**
Erfinder: **Denke, László, Dipl.- Ing., Solyom ut 16/B, H-1126 Budapest (HU)**
Erfinder: **Rideg, György, 2870 Kisbér, Muskátli utca 2 (HU)**
Erfinder: **Kovacs, Kálmán, Dr. Diplomwirt, Kassa Köz 7/a, H-1142 Budapest (HU)**
Erfinder: **Csaplinszky, László, Dipl.- Ing., Csalogány ut 54, H-2030 Erd (HU)**
Erfinder: **Igazvölgyi, Csaba, Dipl.- Chemie- Ing., Szövö ut 22, H-9000 Györ (HU)**
Erfinder: **Wappel, Kálmán, Dipl. Chemie- Ing., Otthon ut 7, H-9000 Györ (HU)**
Erfinder: **Füzy, Péter, Rákoczi ut 91, H-9343 Beled (HU)**
Erfinder: **Lehotzky, Frau György, Dipl.- Ing., Mádi ut 91, H-1104 Budapest (HU)**
Erfinder: **Török, Pál, Dipl.- Ing., Balogh Adám ut 9, H-1026 Budapest (HU)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

## Beschreibung

Die vorliegende Erfindung betrifft eine Anlage mit aus synthetischer Persenning hergestellten Behältern zur schrittweisen (aeroben und anaeroben) Gärung von tierischem Streumist und landwirtschaftlichen und/oder lebensmittelindustrieellen Nebenprodukten, wobei der Füllvorgang der Behälter vorteilhafterweise durch Abkippen vom Transportwagen erfolgt und die Entleerung aus den Gärbehältern pneumatisch durchgeführt werden kann. Die Anlage dient zur Erzeugung von Biogas und Biodünger, der nährstoffreich in herkömmlicher Konsistenz ist und eine ausgezeichnete Qualität hat.

Bekannt ist, daß gut ausgereifter Dünger aus tierischem Streumist und landwirtschaftlichen - eventuell lebensmittelindustriellen - Nebenprodukten als Dünger von hoher Qualität durch das sogenannte halbnasse Gärungsverfahren erzeugt wird, bei welchem auch Biogas entsteht. Dieser Arbeitsvorgang wurde mittels Anlagen durchgeführt, die anfangs mit Betongärbehältern ausgerüstet waren, welche später durch Stahlgärbehälter und Stahlgasbehälter mit Wassersperre ersetzt wurden.

Solche Lösungen sind z.B. in den folgenden Literaturstellen erwähnt.

1. M. Ismann: Biogas, Versuchsstelle in Boineville, neue Anlage: Comptes Rondus des Seances de l'Academia d'Agriculture de France, Paris 1979, Nr. 5, Seiten 415-424.

Entsprechend dieser Veröffentlichung wurde das in zwei Schritten erfolgende halbnasse Verfahren 1974 in Frankreich weiter entwickelt, wonach das Material nach einer äußeren aeroben Vorgärung in einen Gärbehälter aus Stahlplatten überführt wurde. Dieser bestand aus einer Bodenplatte und einem darauf angeordneten Luftgebläse, einer dreiseitig festgelegten und an einer Seite bewegbaren Seitenwand sowie aus einer abschließbaren Dachkonstruktion.

Der Nachteil dieser Anlage besteht darin, daß der technologische Arbeitsvorgang aus zwei Schritten und an zwei Stellen mit zweimaliger Beladung durchzuführen ist. Die gasdichte Ausbildung mit einer mobilen Seitenwand ist dabei schwierig und unsicher.

2. M. Ismann: Sprechen wir nochmals Über das Biogas, Motorisation et Technique Agricole, Paris 1981, Nr. 30, Seite 25.

Der aus Ställen gewonnene Mist wird in einen ihn verflüssigenden Metallbehälter geleitet, in welchem das im Mist befindliche Trockenmaterial durch eine Mischpumpe zerstückelt wird. Die zugegebene Flüssigkeit ist vorzuwärmen. Nach dem Hereinpumpen in den Gärbehälter wird die Mistbrühe abgeleitet, wodurch das Trockenmaterial - mit einem entsprechenden Feuchtigkeitsinhalt - in dem Gärraum erwärmt zurückbleibt. Vom Boden wird dann der Biodünger mittels einer Schneckenpumpe herausgehoben. Der Nachteil dieser Lösung liegt darin, daß die Flüssigkeit infolge der Zuleitung von Feuchtigkeit früher oder später derart angereichert wird, daß sie ausgetauscht werden muß. Dies ist ein gefährlicher und dabei auch ein Wärmeverlust verursachender Vorgang. Außerdem verursacht die gasdichte Ausbildung der für das Herausheben verwendeten Schneckenpumpe ein unsicheres Gashalten.

3. W. Wujcik und W-J. Jewel: Anaerobe Halbtrockengärung, Biotechnology and Bioengineering Symposion, No. 10 (1980), Cornell University, Ithaca.

In dieser Literaturstelle ist die technologische Untersuchung der anaeroben Gärung und deren Auswertung beschrieben, die gleicherweise positive wie auch negative Ergebnisse aufweist.

Nach den Ergebnissen dieser Untersuchung sind die üblichen Formgestaltungen des Metallbehälters als negativ zu bezeichnen, da sie in Anbetracht der Materialförderung kritisch und in bezüglich der Parameter der eingegebenen Materialien nachteilig sind.

Bei den auf diese Weise ausgestalteten, Biogas und Biodünger erzeugenden Halbtrockengärungsverfahren wird der Rohstoff nach dessen Förderung an die Vorgangsstelle früher meistens durch manuelle Abladung, später mittels einer Abladmaschine entladen. Heutzutage aber erfolgen bei den herkömmlichen Gärbehältern die Beladung des Rohstoffes und die Entleerung des Biodüngers durch in der Landwirtschaft gebräuchliche Traktor-Schiebeplatten, Mistgreifern, bzw. Kombinationen derselben. Auf diese Weise wurde hinsichtlich des Be- und Entladens ein bedeutender technischer Fortschritt erzielt, da sich dadurch die Materialbeförderung in den landwirtschaftlichen Produktionseinheiten unterschiedlicher Größe zur Erzeugung von Biogas und Biodünger besser den Anforderungen des Umweltschutzes anpassen ließ; jedoch war ein derartiges Verfahren nur in mehreren Schritten durchführbar.

Hierzu waren im einzelnen folgende Arbeitstakte erforderlich:

- die Beladung eines Beförderungsfahrzeuges mit Rohstoff und dessen Förderung zum Zielort;
- die Nachanpassung des beladenen Rohstoffes entsprechend der Formgestaltung der Gäranlage was entweder maschinell oder notfalls manuell erfolgte;
- das Herausheben des ausgereiften Materials aus dem Gärbehälter und Beladen des Transportfahrzeuges damit mittels eines Greifzuges;
- nach dem Herausheben das manuell erfolgende Zusammenkratzen, Herausheben und Weiterfördern des maschinell nicht heraushebbaren zurückgelassenen Restes.

Ziel der vorliegenden Erfindung ist es, eine Anlage zur Erzeugung von Biogas und Biodünger aus tierischem Streumist und landwirtschaftlichen Nebenprodukten zu schaffen, bei der mit Wasser in im vorbestimmten Verhältnis zu wesentlichen Mengen aus großbetrieblicher Viehhaltung stammendem dünnflüssigen Dünger verwendet wird und die im Vergleich mit dem üblichen, in zwei Schritten erfolgenden halbtrockenen (aeroben und anaeroben) Verfahren und den zu deren Durchführung üblichen Anlagen verbessert ist und durch die der Bedarf an Rohstoffen und der Energieaufwand z.B. Wärmeenergie, der Bedarf für deren Zuführung und wie auch der Energieaufwand für die Entleerung des Biodüngers wesentlich herabgesetzt wird.

Mit dem erfindungsgemäßen Verfahren wird Energie produziert, so daß weniger Fremdenergie für die Heizung und Materialbehandlung erforderlich ist.

Die Zielsetzung der erfindungsgemäßen Anlage liegt darin, anstatt der bisherigen stationären Anlagen vom unterschiedlichen Typ eine wesentlich einfacher betreibbare, mobil anlegbare und hinsichtlich des Investitionsbedarfes kostengünstigere Lösung zu schaffen, bei der die wichtigsten Bestandteile der Anlage als typisierte serienmäßig herstellbare Vorverfertigungsteile ausgelegt sind, wodurch wiederm höhere Qualitätsanforderungen erzielbar sind.

Eine weitere Zielsetzung der Erfindung liegt darin, anstatt der bei den bisherigen, in zwei Schritten erfolgenden technologischen Verfahren, bei denen voneinander getrennte stabile Behälter verwendet wurden, einen einzigen, teilweise mobilen Kunststoffbehälter sowohl für den aeroben als auch für den anaeroben Arbeitsvorgang zu verwenden.

Der größte Teil dieser Gärbehälter von unterschiedlicher (hauswirtschaftlicher, mittel- und großbetrieblicher) Größenordnung für das in zwei Schritten erfolgende Verfahren läßt sich mit vorgefertigten und aus synthetischer Persenning hergestellten Elementen durch eine schnelle und nicht aufwendige Verbindung zu einem am Bestimmungsort eingebauten herkömmlichen Konstruktionselement in der erfindungsgemäßen Weise ausbilden. Der an dem Gärbehälter angeschlossene Gasbehälter wird anstatt aus dem herkömmlichen Baumaterialen auf ähnliche Weise aus vorteilhafterweise ebenfalls synthetischer Persenning hergestellt, so daß ein besonderer Korrosionsschutz, bzw. ein sehr energiebedürftiger Schutz gegen Einfrieren wie bei den herkömmlichen mit einer Wassersperre versehenen Anlagen entfällt.

Der Erfindung liegt ferner die Erkenntnis zugrunde, daß heutzutage schon solche synthetischen Materialien zur Verfügung stehen, die sich zum Behälterbau, bsp. zu aus Persenningen bestehenden Behältern, verarbeiten lassen, die über große Temperaturbereiche, beispielsweise -30°C bis +80°C gasdicht sind. Als geeignete Materialien werden dabei solche Stoffe verwendet, die über die gewünschten mechanischen Eigenschaften gewährleistende, synthetische Gewebeträger sind und eine Gasdichte, chemische Widerstandsfähigkeit gewährleistende, beidseitig auf das Gewebe aufgebrachte Filmschicht aus Kunststoff haben, die im zusammengeschweißten Zustand den bei einem in zwei Schritten erfolgenden halbtrockenen technologischen Verfahren zur Erzeugung vom Biogas und Biodünger in unterschiedlichen Größenordnungen entstehenden Belastungen sicher standhalten, und dabei leicht konfektonierbar sowie durch Verschweißen kraftbeständig verbindbar und im Falle einer Beschädigung einfach reparierbar sind.

Trotz der für die Verfahren zur Erzeugung vom Biogas und Biodünger in Betracht gezogenen Rohstoffe, bleibt der erfindungsgemäße Behälter aufgrund des verwendeten Materials hinsichtlich seiner Standzeit und seiner Beanspruchbarkeit und seiner technischen Eigenschaften des Gärungsvorganges voll funktionstüchtig und beständig. Aus der vorgenannten gasdichten synthetischen Persenning als Baumaterial lassen sich Gärbehälter, Trockengasbehälter sowie Elemente für Biodüngerbehälter mit wählbarem Volumen werkseitig in unterschiedlichen typisierten Größen herstellen. Die derartig hergestellten Behälter können dann noch entsprechenden Qualitätskontrollen in einem Zustand von hoher Qualität an den Bestimmungsort in-"hauswirschaftlichen oder betrieblichen" Größen geliefert und dort aufgestellt oder aufgebaut werden. Dabei werden den Behältern die für die herkömmliche Betriebsart notwendigen weiteren technologischen Elementen zugeordnet, so daß auf diese Weise aus diesen eine komplette Anlage zur Erzeugung vom Biogas und Biodünger entstehen.

Die vorliegende Erfindung betrifft also eine Anlage mit aus synthetischer Persenning hergestellten Behältern zur schrittweisen aeroben und anaeroben Gärung von tierischem Streumist und landwirtschaftlichen und/oder lebensmittelindustriellen Nebenprodukten, wobei der Anlage Biodauchezuführer, Vorwärmekessel, Pumpen, sowie Materialbeförderungsorgane mit einem Verdichter zugeordnet sind. Die Biojauchezuführer sind mit den Gärbehältern verbunden, die wiederum über eine Leitung mit einem Trockengasbehälter verbunden sind. Außerdem besitzt die Anlage auch Biodüngerdiemen.

Das Wesen der vorliegenden Erfindung liegt darin, daß die Anlage mit einer für aerobe Prozesse dienenden, gasdichten, synthetischen Persenning ausgerüstet ist und zumindest ein Gärbehälter vorgesehen ist, der von einer Wassersperre umgeben ist. Ferner ist der Unterteil des Gärbehälters aus einer unteren synthetischen Persenning, einer mittleren synthetischen Persenning und einer oberen löcherigen synthetischen Persenning gebildet, wobei zwischen der mittleren synthetischen Persenning und der unteren synthetischen Persenning ein den über der oberen löcherigen synthetischen Persenning befindlichen Dünger herauskippendes Luftgebläseorgan eingefügt ist.

Ferner ist zwischen der oberen löcherigen synthetischen Persenning und der mittleren synthetischen Persenning eine die aerobe Behandlung des Düngers versorgende Luftleitung eingebaut. Außerdem verfügt der Gärbehälter über eine synthetische Dachpersenning, die an ihrer einen Seite befestigt, an ihren anderen drei Seiten jedoch vorteilhafterweise mit Ösen versehen ist, durch die ein Lösen und Befestigen des bodennahen Rands der Dachpersenning ermöglicht ist. Die Dachpersenning wird durch eine als Hebeorgan ausgebildete Winde hochgehoben oder abgesenkt.

Eine vorteilhafte Ausführungsform der Erfindung besteht darin, daß ein Teil der synthetischen Dachpersenning mit einer die Zuführung der Jauche gewährleistenden löchrigen synthetischen Dachpersenning versehen ist so daß dieser Dachteil doppelwandig ausgebildet ist.

Eine weitere bevorzugte Ausführungsform der Erfindung besteht darin, daß das Speicherorgan für den ausgereiften Dünger an einer aus gasdichter synthetischer Persenning hergestellten Konstruktion gebildet ist, die mittels lösbarer Haken befestigt ist.

Eine beispielhafte Ausführungsform der vorliegenden Erfindung wird anhand der Zeichnung eingehender beschrieben. In der Zeichnung zeigt:

Figur 1 die Anordnung der erfindungsgemäßen Anlage,

Figur 2 die Konstruktion eines einzelnen erfindungsgemäßen Gärbehälters sowie

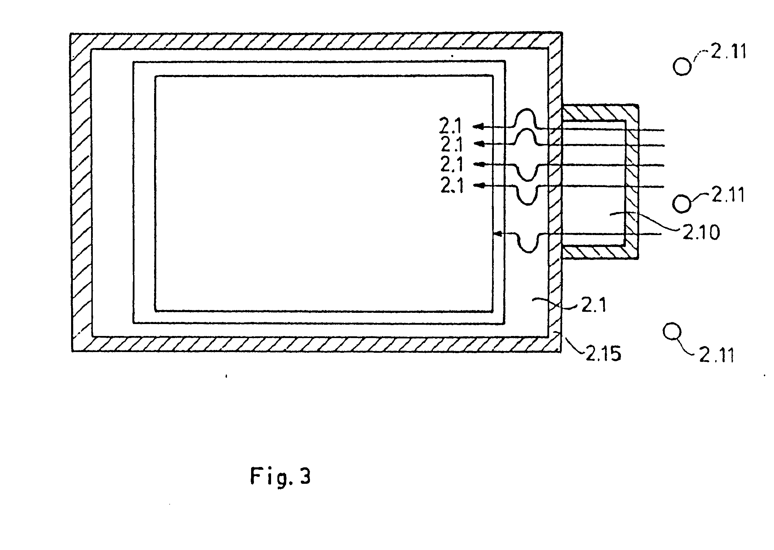

Figur 3 die Draufsicht des aus Figur 2 ersichtlichen Gär: behälters.

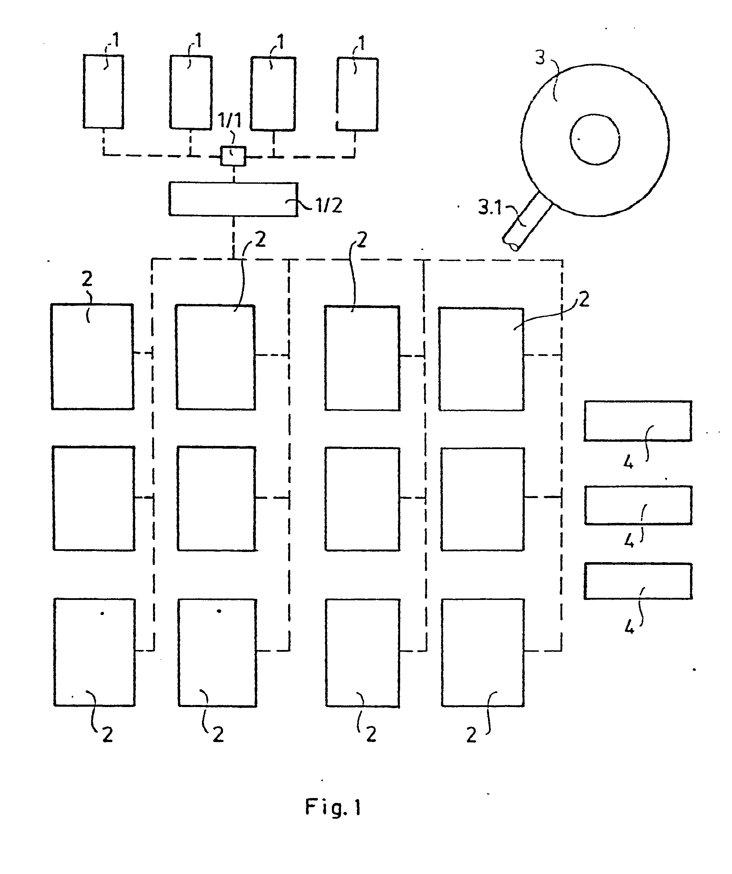

Wie aus Fig 1 ersichtlich, sind mehrere Biojauchezuführer 1 mit einem Vorwärmkessel 1/1 mit üblicher Feuerung, sowie Pumpen und Verdichter 1/2 enthaltenden mobilen Einrichtungen versehen. Die aus synthetischer Persenning, beispielsweise aus einem starkfädigen, wasserdicht ausgerüsteten Chemiefaser-Segeltuch in Leinwand- und doppelfädiger Leinwandbindung, hergestellten Gärbehälter 2 sind mit einem Mechanismus zum Aufziehen des Persenningzeltes versehen, z.B. einer Winde 2.11, und mit an ihn angeschlossenen Röhren sowie mit einem Schacht für die Meßinstrumente versehen. Ein gewichtsbelasteter Trockengasbehälter 3 ist vorteilhafterweise mittels eines Luftkissens wärmeisoliert und über eine Leitung 3.1 an Durchleitungsrohre 2.16 angeschlossen, die in den Biogasraum münden. Zu der erfindungsgemäßen Anlage gehören ferner Biodüngerdiemen 4, die vorteilhafterweise aus gasdichter synthetischer Persenning derart hergestellt sind, daß sie für ihre Beladung und Entleerung durch eine Schobermaschine mit entsprechenden Haken ausgerüstet sind.

Die einzelnen Teile der vorliegenden Anlage sind durch übliche Rohrleitungen miteinander verbunden.

Eine Gruppe der erfindungsgemäßen Gärbehälter 2 mit einem in zwei Schritten erfolgenden technologischen Arbeitsvorgang (siehe Fig 1) gewährleistet eine kontinuierliche Verarbeitung des Rohstoffes. Die Größenordnung des täglich zu verarbeitenden Rohstoffes, bzw. der sich daran anpassend erzeugten Energie bestimmt die Größenordnung je eines Gärbehälters, die bei einer erfindungsgemäßen Anlage für die Hauswirtschaft circa $V = 10m^3$ beträgt und für betriebliche Verhältnisse zwischen $V = 100\text{-}200\ m^3$ ein beliebiges Volumen aufweisen kann.

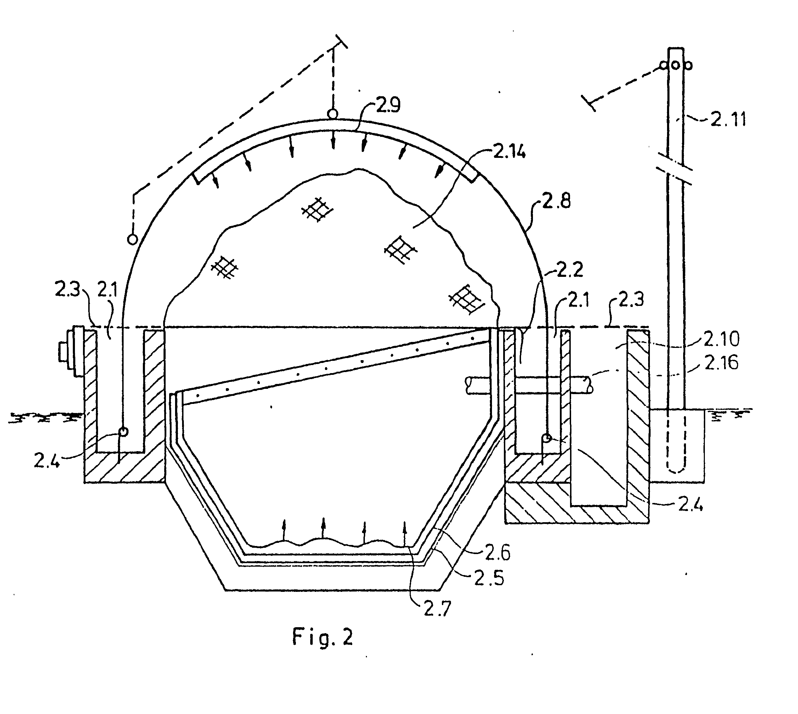

Jeder Gärbehälter 2 besteht im wesentlichen aus einer Wassersperre 2.1 mit rechteckigem Grundriß, an dessen Schmalseiten eine zum Innern des Gärbehälters hin schrägverlaufende Bodenwand angrenzt (siehe Fig 2).

Die obere freie Öffnung der Wassersperre 2.1 ist durch wärmeisolierte Abdeckbohlen 2.3 zugedeckt, die an der Seitenwandung der Wassersperre 2.1 gelenkig angeschlossen sind, während auf der Bodenplatte der Wassersperre 2.1 in einem Abstand von durchschnittlich 30 cm Haken angeordnet sind, die in die Ösen der synthetischen Dachpersenning 2.8 einsteckbar sind. Die Wassersperre 2.1 kann aus Kunststoff, Stahl, oder auch aus am Ort hergestellten Stahlbeton bestehen. Die Wassersperre 2.1 ist in der Regel rings um die untere synthetische Persenning 2.5, vorteilhafterweise in vorgefertigter Form angeordnet.

In einem Schacht 2.10 sind die an den Gärbehälter 2 angeschlossenen, die Biojauche ableitenden sowie die die Biojauche durch Preßluft zurückleitenden Leitungen untergebracht, die zu der in zwei Schritten erfolgenden (aeroben) Erwärmung des Rohstofes notwendig ist. Die Preßluft wird zwischen der unteren synthetischen Persenning 2.5 und der mittleren synthetischen Persenning 2.6 zum Umwenden der mittleren synthetischen Persenning 2.6 eingeblasen, wobei die das entstandene Biogas ableitenden Rohrleitungen mit ihren Armaturen in der Zeichnung zur besseren Obersicht nicht dargestellt sind.

Nach dem bekannten und in der Regel auch erfolgreich betriebenen Halbtrockengärungsverfahren wird für die erfindungsgemäße Anlage nur ein einziger Gärbehälter aus synthetischer Persenning verwendet, der für die beiden technologischen Phasen, d.h. für die aerobe und anaerobe Phase, dient, wodurch die Mechanisierung hinsichtlich Beladung und Entleerung vereinfacht, und beschleunigt und gegenüber den bisher weltbekannten Lösungen hinsichtlich des Energieaufwandes wesentlich günstigere Ergebnisse erzielt werden, da hier die Beladung mit Rohstoff durch Umkippen des durch einen Traktor geschleppten Anhängers erfolgt, der auf dem an dem Gärbehälter befindlichen Verkehrsweg an die Gärbehälter herangefahren wird und von dem der Rohstoff unmittelbar auf die aus unterer synthetischer Persenning 2.5, mittlerer synthetischer Persenning 2.6 und oberer löchriger synthetischer Persenning 2.7 bestehenden Schicht gelangt, wodurch beim Einfüllen gleichzeitig ein entsprechendes Vermischen der Rohstoffe gewährleistet ist.

Die Einfüllung erfolgt in Form eines Halbkreises, d.h. kegelförmig, wobei in je einen Gärbehälter 2 nur eine Füllung (1-6 Tage) Rohstoff möglichst innerhalb einer Füllungszeitdauer von 8-10 Stunden gelangt. Die Einstellung des entsprechenden Feuchtigkeitsgehaltes des Rohstoffes während der Füllung erfolgt derart, daß die aus den vorangegangenen Füllungen schon in den Schacht 2.10 abgelassene biologisch aktive Jauche mittels einer Pumpe auf die auf der oberen löchrigen synthetischen Persenning 2.7 befindlichen Rohstoffladung 2.14 geleitet wird.

Zur gleichen Zeit, wenn der Füllungvorgang fest abgeschlossen ist, wird aus dem Schacht 2.10 Preßluft in einer Menge von 0,5-1,6 m³/m³h zwischen der mittleren synthetischen Persenning 2.6 und der oberen löchrigen synthetischen Persenning 2.7 eingeblasen, um dadurch eingelagerten Rohstoff auf aerobe Weise so schnell wie möglich unter Mitwirkung von Sauerstoff benötigende Bakterien derart zu erwärmen, daß die Füllung eine Durchschnittstemperatur von +60°C erreicht.

Wenn die eingebauten, jedoch in der Zeichnung nicht dargestellten Fernthermometer das Erreichen der Temperatur von +60°C anzeigen, zieht das Bedienungspersonal das aus synthetischer Dachpersenning 2.8 und synthetischer löchriger Dachpersenning 2.9 hergestellte Zelt auf und befestigt dessen bodennahe Ränder mittels den Ösen 2.4 in der Wassersperre 2.1. Dann verbindet das Bedienungspersonal in dem Schacht 2.10 sämtliche durch die Wassersperre 2.1 hindurchgehende Ein- und Ausleitungsrohre. Anschließend wird die synthetische Dachpersenning 2.8 verschlossen und die Wassersperre 2.1 aufgefüllt. Durch die synthetische, löchrige Dachpersenning 2.9 läßt sich die Biojauche auch während des Arbeitsvorganges (im Betrieb) auf die im gasdicht abgeschlossenen Gärbehälter 2 befindliche Rohstoffladung 2.14 aufbringen, um so deren Feuchtigkeitsgrad zu erhöhen bzw. den pH-Wert der Rohstoffladung 2.14 eventuell zu verändern, wobei die erforderlichen Neutralisationsmittel der Biojauche im voraus beigefügt werden.

Nach einer ausnutzbaren Periode von ca. 20-35 Tagen sperrt das Bedienungspersonal die zum Gasbehälter führende Gasleitung ab und leitet das noch entstehende Gas in eine ins Freie führende Leitung ein, wodurch der Druck im Inneren des unter Betriebdruck stehenden Gärbehälters 2 abfällt. Dadurch können die Ösen 2.4 der synthetischen Dachpersennin9 2.8, - die als ein Dachzelt ausgebildet sein kann von der Wassersperre 2.1 enthakt und die Dachpersenning mittels der Winde 2.11 derart hochgehoben werden, daß die ausgereifte Rohstoffladung 2.14 an der Seite des Verkehrsweges völlig frei wird.

Die sich auf der mittleren synthetischen Persenning 2.6 angesammelte Flüssigkeit (Jauche) kann durch die Ableitungskanäle und den Schacht 2.10 in den Pumpenschacht des Dispatchergebäudes geleitet werden.

Zwischen der unteren synthetischen Persenning 2.5 und der mittleren synthetischen Persenning 2.6 wird dann Preßluft eingeleitet. Dadurch wird der Innenraum der oberen löchrigen synthetischen Persenning von dem einen Ende in die Richtung des anderen Endes des Gärbehälters 2 mittels Luft aufgefüllt, wodurch gleichzeitig die im Gärbehälter 2 befindliche ausgereifte flüssigkeitslose Rohstoffladung 2.14 auf den Verkehrsweg hochgehoben wird. Von hieraus wird sie auf übliche Weise, z.B. durch Beladung von Traktorenanhängern (Schiebeplatte und Greifer) in den Biodüngerspeicher gefördert.

Mittels einer Takelage, die über die auf Säulen befindlichen Rollen der Winden 2.11 läuft, wird die synthetische Dachpersenning 2.8 hochgehoben. Die Winde 2.11 hat vorteilhafterweise einen elektrischen Antrieb, sie kann jedoch auch manuell betätigt werden.

**Patentansprüche**

1. Anlage mit aus synthetischer Persenning hergestellten Gasbehältern zur schrittweisen (aeroben und anaeroben) Gärung von tierischem Streumist und landwirtschaftlichen und/oder lebensmittelindustriellen Nebenprodukten, mit Biojauchezuführer, denen Vorwärmkessel, Pumpen sowie Materialbeförderungsorgane mit Verdichter zugeordnet sind, wobei die Biojauchezuführer mit den Gärbehältern verbunden sind, die ihrerseits über eine Leitung mit einem Trockengasbehälter verbunden sind, wobei die Anlage auch Biodüngerdiemen aufweist, dadurch gekennzeichnet, daß die Anlage mit für aerobe Prozesse dienenden, gasdichten, synthetischen Persenningen ausgerüstet ist und daß zumindest ein Gärbehälter (2) vorgesehen ist, der von einer Wassersperre (2.1) umgeben ist, daß der Unterteil des Gärbehälters (2) von einer unteren synthetischen Persenning (2.5), einer mittleren synthetischen Persenning (2.6) und einer oberen löchrigen synthetischen Persenning (2.7) gebildet ist, daß zwischen der mittleren synthetischen Persenning (2.6) und der unteren synthetischen Persenning (2.5) ein den auf der oberen löchrigen synthetischen Persenning (2.7) liegenden Dünger herauskippendes Luftgebläseorgan angeordnet ist, daß zwischen der oberen löchrigen synthetischen Persenning (2.7) und der mittleren synthetischen Persenning (2.6) eine die aerobe Behandlung des Düngers versorgende Luftleitung eingebaut ist, daß ein die obere Folienabdeckung des Gärbehälters (2) bildendes Dach als synthetische Dachpersenning (2.8) vorgesehen ist, die an ihrer einen Seite befestigt, an ihren anderen drei Seiten vorteilhafterweise über Ösen (2.4) auf- und zuschließbar ist und daß als Hebeorgan der auf- und zuschließbaren Seiten der synthetischen Dachpersenning (2.8) eine Winde (2.11) vorgesehen ist.

2. Anlage nach Anspruch 1, dadurch gekennzeichnet, daß ein Teil der synthetischen Dachpersenning (2.8) mit einer die Zuführung der Jauche gewährenden löchrigen synthetischen Dachpersenning (2.9) versehen ist, so daß dieser Dachteil doppelwandig ausgebildet ist.

3. Anlage nach Anspruch 1 oder 2, dadurch gekennzeichnet, daß das Speicherorgan für den ausgereiften Dünger von einer aus gasdichter synthetischer Persenning hergestellten Konstruktion gebildet ist, die mittels lösbarer Haken befestigt ist.

## Claims

1. A plant with gas containers made of synthetic tarpaulin for the progressive (aerobic and anerobic) fermentation of animal manure and or agricultural and/or the food industry byproducts, comprising a feeder of biological liquid manure with which a preheat-boiler, pumps as well as material transport devices with compressor are associated, wherein the feeder of biological liquid manure is connected with the fermentation vessels which in turn are connected via a pipe to a dry gas container, the plant also including biological manure storage stacks, characterised in that the apparatus is equipped with gas-tight synthetic tarpaulins for aerobic processes; and that there is provided at least one fermentation vessel (2) surrounded by a water-lock (2.1); that the lower part of the fermentation vessel (2) is formed by a lower synthetic tarpaulin (2.5), by a middle synthetic tarpaulin (2.6) and a perforated upper synthetic tarpaulin (2.7); that an air blower device is arranged between the middle synthetic tarpaulin (2.6) and the lower synthetic tarpaulin (2.5) for ejecting manure lying on the upper synthetic tarpaulin (2.7); that an air supply pipe for the aerobic treatment of the manure is installed between the upper perforated tarpaulin (2.7) and the middle synthetic tarpaulin (2.6); that there is provided a lid forming the upper foil covering of the fermentation vessel (2) in the form of a synthetic tarpaulin lid (2.8) which is fixed on one side and on the other three sides is openable and closable, expediently by way of eyelets (2.4), and that a winch (2.11) is provided as the raising device for the openable and closable sides of the synthetic tarpaulin lid (2.8).

2. A plant according to claim 1, characterised in that one part of the synthetic tarpaulin lid (2.8) is provided with a perforated synthetic tarpaulin lid (2.9) for assuring the feed of the manure, so that this part of the roof is double-walled.

3. A plant according to claim 1 or claim 2, characterised in that the storage device for the matured manure is constructed as a gas-tight synthetic tarpaulin structure which is rastened by releasable hooks.

## Revendications

1.- Installation comportant des récipients à gaz formes de toile à bâche synthétique pour la fermentation étagée (aérobie et anaérobie) de fumier animal d'épandage et de sous-produit agricoles et/ou de l'industrie alimentaire, comportant des conduits d'amenée de purin biologique associés à des cuves de préchauffage, des pompes et des organes transporteurs de matériau munis d'un compacteur, les conduits d'amenée de purin biologique étant reliés aux cuves de fermentation qui sont reliés à leur tour, par un conduit à un récipient à gaz sec, l'installation comportant par ailleurs des reservoirs d'engrais biologique, caractérisé en ce que l'installation est équipée de toiles à bâche synthétiques, imperméables aux gaz, qui sont utilisées pour des processus aérobies, en ce qu'il est prévu au moins une cuve de fermentation (2) qui est entoure d'un barrage d'eau (2.1), en ce que la partie inférieure de la cuve-de fermentation (2) est formée d'une toile à bâche synthétique inférieure (2.5), d'une toile à bâche synthétique médiane (2.6) et d'une toile à bâche synthétique supérieure perforée (2.7), en ce qu'un organe de soufflage d'air evacuant par basculement l'engrais placé sur la toile à bâche synthétique supérieure perforée (2.7) est disposé entre la toile à voiles synthétique médiane (2.6) et la toile à bâche synthétique inférieure (2.5), en ce qu'un conduit à air assurant l'alimentation en air intervenant dans le traitement aérobie de l'engrais est disposé entre la toile à bâche synthétique supérieure perforée (2.7) et la toile à bâche synthétique médiane (2.6), en ce qu'il est prévu un toit sous forme de toit en toile à bâche synthétique (2.8) qui constitue une feuille de recouvrement supérieure de la cuve de fermentation (2) et qui est fixée sur un de ses côtés et susceptible d'être ouverte ou fermée, de préférence au moyen d'oeillets (2.4), sur ses trois autres côtés, et en ce qu'un organe de levage des côtés de la toile à bâche synthétique formant toit (2.8) et susceptibles d'être ouverts ou fermés est constitué par un treuil (2.11).

2.- Installation selon la revendication 1, caractérisée en ce qu'une partie de la toile à bâche synthétique formant toit (2.8) est munie d'une toile à bâche synthétique perforée de toit (2.9) permettant l'amenée du purin, de sorte que cette partie du toit présente une double paroi.

3.- Installation selon la revendication 1 ou 2, caractérisée en ce que l'organe de stockage de l'engrais fermenté est formé d'une structure de toile à bûche synthétique imperméable aux gaz qui est fixée au moyen de crochets amovibles.

Fig. 1

Fig. 2

Fig. 3